(19) Europäisches Patentamt
European Patent Office
Office européen des brevets

(11) **EP 4 462 119 B1**

(12) **EUROPEAN PATENT SPECIFICATION**

(45) Date of publication and mention
of the grant of the patent:
**11.03.2026 Bulletin 2026/11**

(21) Application number: **22859518.7**

(22) Date of filing: **13.12.2022**

(51) International Patent Classification (IPC):
**G01N 33/493** (2006.01)

(52) Cooperative Patent Classification (CPC):
**G01N 33/493**

(86) International application number:
**PCT/ES2022/070796**

(87) International publication number:
**WO 2023/111375 (22.06.2023 Gazette 2023/25)**

(54) **AUTOMATIC URINE MEASURING DEVICE**

AUTOMATISCHE URINMESSVORRICHTUNG

DISPOSITIF DE MESURE URINAIRE AUTOMATIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.12.2021 ES 202132437 U**

(43) Date of publication of application:
**13.11.2024 Bulletin 2024/46**

(73) Proprietor: **JUNGLE VENTURES SL**
**08015 Barcelona (ES)**

(72) Inventors:
• **MASÓ, Josep**
**08015 Barcelona (ES)**
• **BLANKING, Pär**
**08015 Barcelona (ES)**
• **FERRÉ, Jordi**
**08015 Barcelona (ES)**
• **CÓNSUL, Sergi**
**08015 Barcelona Barcelona (ES)**
• **VEA, Daniel**
**08015 Barcelona Barcelona (ES)**
• **ARNASTE, Adrià**
**08015 Barcelona Barcelona (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
EP-A1- 3 702 781        WO-A1-2015/196254
WO-A1-2016/176603      WO-A2-2004/036343
KR-A- 20190 012 392     US-A1- 2020 390 422
US-B1- 10 383 606

**Description**

**OBJECT OF THE INVENTION**

The present invention belongs to the field of urinary analysis devices, and particularly to a measuring device for taking urine samples, measuring several parameters of the sample, and recording the measurements, configured to be arranged in a urinary receptacle.

**BACKGROUND OF THE INVENTION**

Biometric parameter analysis and monitoring is a fundamental aspect for the early detection of different health problems, as well as for the performance of continuous follow-ups. Blood analysis stands out among the most commonly used reference methods. Although blood extractions can be performed for subsequent blood measurement, they are invasive, require the intervention of a professional, and are not practical for frequent implementation. In fact, waste from blood and excess blood are removed through urine, serving in turn as means for regulating the balances of different components in the body, such as electrolytes and water. Urine analysis or urinalysis is therefore a convenient way to detect and control different diseases by means of detecting solutes therein. Given that a large amount of molecules, such as glucose, hormones, and derivatives from different biological processes are discharged from the body through urine, urinalysis is a highly effective, non-invasive way to monitor the condition of a subject in multiple aspects, such as hydration, creatine, or glucose levels. Urinalyses can be performed by measuring the amounts of different solutes in urine, analyzing physical properties thereof, such as color, smell, taste, viscosity, conductivity, and pH, among others.

However, current urinalyses are based on either colorimetric analysis, requiring the use of and continuous spending on consumables, or on the use of complex laboratory analysis methods such as mass spectrometry, involving expensive equipment. In this sense, in order to perform analyses, patients are usually required to travel to a center where tests will be performed on said patients, to urinate into a container, and to deliver the sample to a technician for subsequent analysis. This involves a series of drawbacks for the patients who have to travel and provide urine samples to a third party, leading to patient discouragement and a lack of recurrence in analyses. Moreover, laboratory technicians are the ones who carry out the tests, requiring the handling of biological samples and the use of advanced instrumentation, involving a cost in terms of time as well as human and technical resources.

There is therefore a need for a urine measuring system that is as minimally invasive as possible for the patient, as simple as possible for the technician, or can be used without the intervention of a technician, with a real-time measurement and a low cost so that it can be implemented on a recurring basis.

WO 2015/196254 discloses an apparatus for performing analysis on a sample of urine whereby the apparatus comprises a funnel and means to measure reflection from the sample.

**SUMMARY OF THE INVENTION**

A first aspect of the invention relates to a urinary measuring device for taking and measuring urine samples according to independent claim 1.

In a preferred embodiment, the funnel comprises a filter that at least partially covers the opening configured to prevent direct urination through the opening.

In another preferred embodiment, the funnel further comprises a gasket in a part of the urinary measuring device, the gasket configured to contact the urinary receptacle for fluidically sealing a space between the urinary measuring device and the urinary receptacle.

In another preferred embodiment, the funnel further comprises an overflow opening configured to inhibit the sample from overflowing from the funnel, and a conduit configured to receive fluid from the overflow opening, with the conduit bypassing the urine collection unit. In an even more preferred embodiment, the funnel further comprises a lid covering the overflow opening configured to prevent the sample from flowing directly through the overflow opening.

In another preferred embodiment, the light emitter and the light sensor are located in the casing for electronics for emitting and receiving light with respect to the urine collection unit at an angle comprised between 120° and 180°.

In another preferred embodiment, the casing for electronics comprises a main body, a cover, and a gasket configured to fluidically seal the interface between the main body and the cover.

In another preferred embodiment, the casing for electronics comprises a transparent protector arranged for protecting the light emitter and the light sensor against the sample splashing from the urine collection unit.

In another preferred embodiment, the urine collection unit comprises a surface arranged for maximizing the reflection of an incident light and which in turn comprises a material selected from one of the materials from the following list: ceramic, crystal, glass, porcelain, metal, silicone, wood, stones such as marble, polymers such as POM or photosensitive resin, as well as a derivative of these materials.

According to the claimed invention, the urinary measuring device further comprises:

> d. A container for collecting fluid overflowing from the urine collection unit, comprising an outlet opening; and
> e. A plurality of electrodes extending inside the container by means of arms.

The device may comprise a voltage source connected between the electrodes configured to apply a voltage signal between the electrodes and a current meter configured to measure the current between the electrodes resulting from a voltage signal applied by the voltage source. The device may comprise an impedance meter connected to the electrodes, the impedance meter configured to measure the impedance of the fluid present in the container between the electrodes.

According to the claimed invention, the outlet opening of the container is configured to allow an outflow smaller than that of the main opening of the funnel. In another preferred embodiment, the container further comprises a side outlet opening, arranged at a greater height than that of the outlet opening and than that of the plurality of electrodes.

In a preferred embodiment, electric cables extend from the arms to the casing for electronics through one or more gaskets, in order to form an electrical connection between the electrodes and the electronics in the casing for electronics.

In a preferred embodiment, the funnel comprises a handle configured to facilitate the extraction of the urinary measuring device by means of an extraction device.

In a preferred embodiment, the casing for electronics further comprises a controller configured to control the light emitter, the light sensor, and optionally the impedance meter, and the controller is made up of a processor configured to process information from the light emitter, light sensor, and optionally from the impedance meter.

In a preferred embodiment, the urinary measuring device further comprises a valve configured to control the outflow of water from a water source to the urinary receptacle, and the device is configured to control the valve. In an even more preferred embodiment, the urinary measuring device is configured to open the valve when it detects that sample measurement has ended.

In a preferred embodiment, the urinary measuring device further comprises a user interface configured to receive identification or anonymous information of a user and to receive information about the urinary sample of the user and to show information about the condition of the sample to the user.

## BRIEF DESCRIPTION OF THE FIGURES

To allow a better understanding of the present disclosure and to show how it can be carried out, reference is now made, only by way of example, to the attached schematic drawings in which:

> Figure 1 shows a perspective view (A) and a profile view with a cross-section (B) of the urinary measuring device according to one or more embodiments.
> Figure 2 shows a filter in a profile view (A), in a perspective view (B), and being placed in a funnel (C) according to one or more embodiments.
> Figure 3 shows an exploded view of the gasket and the funnel (A) and a plan view of a gasket (B) according to one or more embodiments.
> Figure 4 show a profile view (A), a plan view (B) of a funnel according to one or more embodiments. Furthermore, it shows a cross-section view (C) of the placement of the funnel with respect to the container according to one or more embodiments.
> Figure 5 shows a plan view (A) and a perspective view (B) of a funnel according to one or more embodiments.
> Figure 6 shows a plan view (A) and a profile view (B) of an optical device according to one or more embodiments. Furthermore, it shows a profile view (C) of the placement of the optical device in a casing for electronics for storing the optical system according to one or more embodiments.
> Figure 7 shows a perspective view (A) and a profile view (B) of a urine collection unit according to one or more embodiments. Furthermore, it shows a profile view (C) of the placement of the urine collection unit with respect to a casing for electronics and another profile view (C) of the interaction of light on the surface of the urine collection unit according to one or more embodiments.
> Figure 8 shows a profile view (A), a plan view (B), and a perspective view (C) of a container according to one or more embodiments.
> Figure 9 shows two embodiments (A and B) of a plan view of a handle according to one or more embodiments.
> Figure 10 shows an exploded view of a urinary measuring device according to one or more embodiments.
> Figure 11 shows a diagram of the relationship of a controller with a solenoid valve and a user interface according to one or more embodiments.

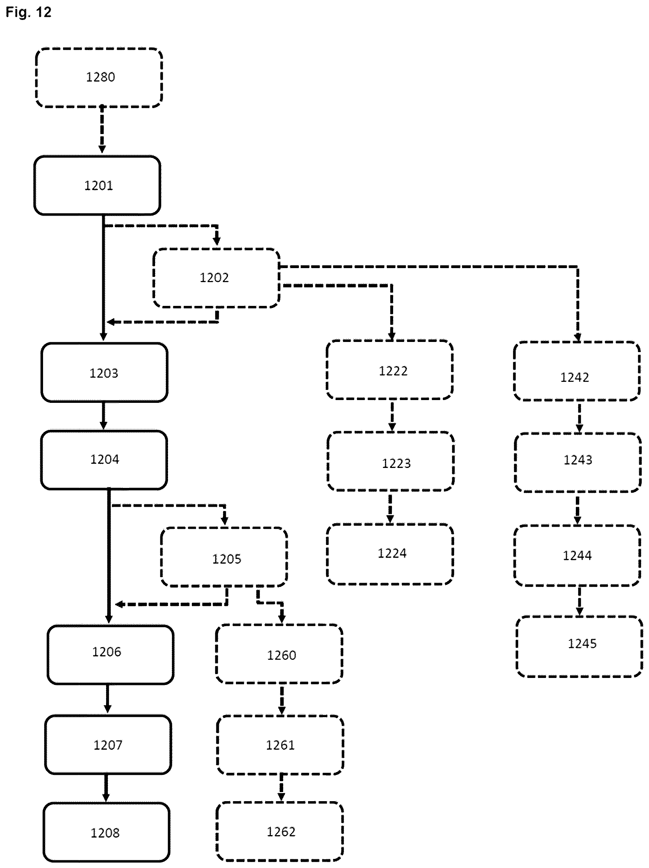

> Figure 12 shows a diagram of the urinary analysis method.
> Figure 13 shows a diagram of an impedance meter.

Figure 14 shows a diagram of a computer program product.

Figure 15 shows a profile view of several optical systems together with their urine collection unit (A, C, and E) together with their respective standard curves (B, D, and F) with respect to a color standard.

## DESCRIPTION OF THE INVENTION

### Definitions

The term "urinary receptacle" refers to any sanitary apparatus adapted to receive and evacuate body waste. In the present invention, a urinary receptacle can be an upright urinal, a wall urinal, a floor urinal, or a urinal adapted for women and/or men, as well as a toilet. A urinary receptacle is unlike a sample holder, such as a bottle, which receives the sample but does not dispose of it such that, after use, the user must discard the sample himself or herself.

The term "funnel" refers to an instrument which allow fluids to pass through a narrower opening from a wide opening. In some embodiments, the funnel allows all the urine sample or water deposited in any part of the funnel to be channeled to a narrower opening through which it is to flow.

The term "main opening" refers to the opening which serves as the narrower opening of a funnel.

The term "urine collection unit" refers to a container for fluid samples which allows containing a fluid sample. In the present invention, the sample is preferably contained such that it can be analyzed by means of a spectroscopy system such as the disclosed optical system. The abbreviated term "UCU" can be used alternatively.

The term "casing" refers to a system formed by at least one part forming a space therein, such that one or more elements can be stored therein. Therefore, the casing for electronics refers to a casing intended mainly for housing electronic elements therein, without being limited to any type of electronic element.

The term "light emitter" refers to an element configured to emit an electromagnetic wave, preferably being a visible or near-visible light (infrared or ultraviolet) emitter.

The term "light sensor" refers to an element configured to measure incident electromagnetic radiation and provide a response indicative of the measured electromagnetic radiation. Preferably, it is a visible or near-visible light (infrared or ultraviolet) sensor.

The term "filter" refers to an element which allows the passage of fluids therethrough, while preventing the passage of unwanted materials such as solids and/or urine bubbles.

The term "gasket" refers to an element configured to prevent or inhibit fluid communication between two spaces.

The term "lid" refers to an element configured to prevent one element from directly accessing (in a straight line) another target element. When the lid covers an opening, the lid is anything that, from a specific position, prevents another element from directly accessing the opening.

The term "protector" refers to an element configured to limit the exposure of another element to an agent. In the case of an optical system, it refers to anything that prevents agents such as lime, dirt, water, or acid, among others, from accessing the optical system. It can therefore be a chemical or mechanical substance and a liquid or solid.

The term "container" refers to an element configured to store other elements therein.

The term "handle" refers to a part of an element which can be gripped by the user or a tool for handling the element.

The term "solenoid valve" refers to an electrically or electronically actuated device for controlling the passage of a fluid therethrough.

The term "software" refers to a computer program or a set of computer programs.

The term "hardware" refers to the set of physical elements comprised in an electronic device. The term "impedance" refers to the opposition to alternating current caused by the combined effect of resistance and/or reactance in a circuit. It will be seen that the devices and methods disclosed herein can be configured to measure admittance instead of impedance and to use equivalent threshold admittance values or machine learning algorithms trained in equivalent admittance data.

### Description

The present invention solves the problems set forth above by means of a urinary measuring device placed in a urinary receptacle, which is capable of taking samples, the user analyzing them *in-situ* and in real time, in an objective, automatic, and self-operated manner, providing measurements rapidly and being able to be reused repeatedly without the use of consumables and without the intervention of a third party at a low cost.

*In-situ* real-time measurements can be achieved as a result of one or more sensors present in the urinary measuring device, which allow the device to analyze different properties of a urine sample.

Due to the configuration of the device, the sample is taken and arranged facing an optical system automatically, so the sample can be measured without having to handle same. Furthermore, through different automatic calibration and cleaning methods, one and the same device can be used by different users without requiring the intervention of a third party, since the device automatically detects the end of each urination, and after analyzing the sample, is capable of eliminating it and calibrating the device for the next user. Furthermore, the typology and configuration of the sensors and the urinary measuring system do not require the constant use of consumables for performing multiple urine analyses either.

The invention relates to a urinary measuring device for taking and measuring urine samples, configured to be arranged in a urinary receptacle; characterized in that it comprises:

> a. A funnel for sample collection, comprising a main opening;
> b. A urine collection unit (UCU) connected to the opening for receiving the sample from the funnel;
> c. A casing for electronics positioned close to the UCU, the casing for electronics containing a light emitter arranged for emitting light on the UCU and a light sensor arranged for measuring the light received from the UCU.

It should be highlighted that the urinary measuring device is configured to be arranged in a urinary receptacle, and therefore, the shape of the device as a whole is configured to be adapted to urinary receptacles of a certain type. However, this should not be considered a limitation thereof, since different shapes of the device described in the present invention can be adapted to different urinary receptacles, such as a toilet or a wall urinal. Urinary receptacle should be understood to mean any type of system which allows collecting urine from a subject for treatment. Therefore, the device of the first aspect of the invention, as well as all the embodiments thereof, are configured to be adapted to upright urinals, wall urinals, ground urinals, urinals adapted for women, as well as toilets. Therefore, all the drawings herein present should be understood as a mere exemplification of the device and not as a limiting feature thereof.

It should be highlighted that the funnel comprises the main opening regardless of the placement of the main opening. It is therefore understood that the main opening can be placed at any point of the funnel. Although the funnels preferably comprise the main opening in the lower part thereof, it is not an essential feature of the present invention. Furthermore, the funnel is configured to pass the sample through the main opening. Although most funnels use ramps to direct fluid samples to the main opening by gravity, some embodiments may use other mechanisms that are capable of directing the samples, such as fluid pumps. Therefore, the funnel can be made up of any element which allows the fluid received in the funnel to be directed preferably to the main opening.

The light emitter and the light sensor form an optical system. The optical system allows emitting light in a range of wavelengths with the light emitter and detecting a frequency spectrum of the light with the light sensor. Therefore, the optical system is capable of emitting light on a sample and detecting the absorption of the light emitted by said sample, i.e., the optical system is capable of taking spectrophotometric measurements. Absorption spectrophotometry is based on the Beer-Lambert law which links the amount of light absorbed by a body with the concentration of said body in a solution and can be detected in the light transmitted through the sample or in the light reflected by the surface of the sample. The following is established for a liquid:

$$\frac{I_1}{I_0} = 10^{-\varepsilon lc} = 10^A$$

where $I_0$ is the incoming light intensity, $I_1$ the outgoing light intensity, $\varepsilon$ the absorptivity of the medium, l the distance traveled by the aqueous medium, c the molar concentration of the body in the solution, and A the absorbance. Therefore, the absorbance of the solution with a wavelength $\lambda$ can be obtained such that:

$$A_\lambda = -log_{10}\left(\frac{I_1}{I_0}\right).$$

Given a known device for which the distance l traveled by the light and the absorptivity of the body $\varepsilon$ are known, the concentration of the body in the medium can be obtained such that:

$$c = \frac{A}{l\varepsilon}.$$

However, to that end, the sample must be arranged such that it is placed between the light emitter and the light sensor. In this sense, the UCU works as a container which allows storing fluid samples deposited in the device and presenting them to the optical system so as to enable the spectrophotometric analysis thereof. It should be noted that the sample can be presented to the UCU in different ways. In some embodiments, the sample in the UCU will be presented such that the emitted light interacts with the sample and is then reflected towards the light sensor. However, other ways of arranging the sample are likewise covered by the present invention. For example, the light emitter and the light sensor can be arranged on opposite faces of the UCU, and the UCU may comprise side faces transparent to the emitted light, such that the light travels linearly between the emitter and the sensor, passing through the sample contained in the UCU.

To measure urine samples, a variety of wavelengths, in that sense, both visible and infrared ranges, including near-, mid-, and far-infrared, allow measuring different types of components in the urine. Therefore, the wavelength of the light emitted by the light emitter can range between 360 nm and 2500 nm, and similarly, the wavelength measured by the light sensor can range between 360 nm and 2500 nm.

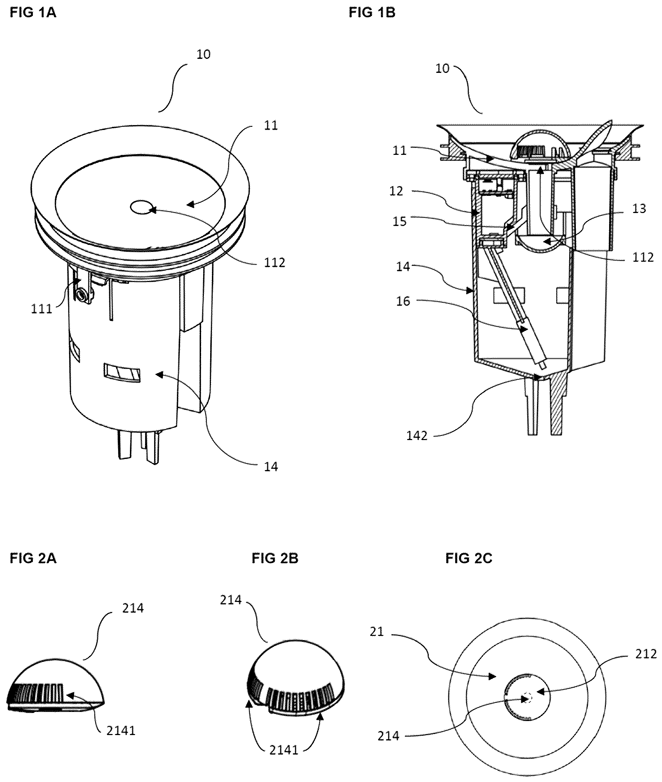

Figure 1 shows a urinary measuring device (10) in a perspective view (Figure 1A) and in a profile view with a cross-section (Figure 1B). It can be seen that the device (10) comprises a funnel (11) with a main opening (112) which allows directing urine samples and water or sensor calibration liquids of the system deposited in the funnel (11) to the main opening (112). The device also has a casing for electronics (12) which contains the electronics and protects same from fluid damage and houses the electronics for an optical **system** (15) for the spectrophotometric measurement of the samples. In this sense, the device also has a urine collection unit (UCU) (13) which allows the samples to accumulate and be arranged such that spectrophotometric measurements can be performed on them by the optical system (15). Lastly, the device has a container (14) which stores excess sample from the UCU (13) and in turn has an outlet opening in its lower part (142) and an electrode system (16). However, in some embodiments, the device does not have the container (14) and/or the electrode system (16).

The perspective view of Figure 1A only shows the funnel (11), the container (14), with the rest of the elements being concealed. Furthermore, it can be seen that the funnel (11) is arranged on the container (14). This embodiment has a fixing element (111) which allows the fixing thereof to other elements, such as the container (14). This fixing element may comprise one or more screws, nuts, openings, flaps, clips, grooves, friction systems, or any other element which allows fixing the funnel to another element of the optical measuring system. However, in other embodiments, the funnel (11) and the container (14) may be in contact. In other embodiments, the fixing element (111) may not be present. Furthermore, the funnel (11) has a circular shape, although in other embodiments the funnel (11) may have other shapes.

In the view with the cross-section of Figure 1B, it can be observed that the funnel (11) provides the sample it collects to the UCU (13) through the main opening (112). Furthermore, it can be seen how the UCU (13) contains the urine sample such that it is exposed to the optical system (15). It can also be observed that, in this particular embodiment, both the casing for electronics (12) and the UCU (13) are contained inside the container (14) and between the funnel (11) and the container (14). However, in other embodiments, the casing for electronics (12) and the UCU (13) may not be contained inside the container (14). For example, the casing for electronics (12) may be located outside the container (14). Lastly, it can be seen that the electrodes (16) are arranged in the container such that they come into contact with the sample contained therein. In other embodiments, the electrodes can be arranged in an alternating manner and at different heights and orientations with respect to one another. Furthermore, the funnel (11) has a channel which guides the sample to the UCU (13) such that splashes are minimized, although in other embodiments the funnel (11) may not have this channel, or the channel may have other shapes.

In some embodiments, the main opening of the funnel has dimensions suitable for depositing urine in the UCU in a controlled manner, such that the formation of foam therein is reduced.

In a preferred embodiment, the funnel comprises a filter that at least partially covers the main opening configured to prevent the user form directly urinating into the UCU through the main opening. The filter is therefore designed to block the direct access of urine, with a reasonable margin from the directions in which urination may take place, so as to cover the different positions that the user may accidentally acquire. Generally, these positions are vertical and front positions, although depending on the urinary receptacle, other positions may be possible, and are therefore object of the present invention. This prevents the entry and the generation of foam. Additionally or alternatively, the filter comprises openings that are small enough so as to prevent the entry of unwanted objects to the main opening, and therefore to the UCU, which may interfere with the sample and the spectrophotometric analysis. Lastly, the filter can be designed with a shape which minimizes the risk of splashing on the user, for example with rounded shapes.

It should be highlighted that, by preventing direct access through the main opening, the speed at which the urine reaches the UCU is significantly reduced, which ensures a reduction of splashes that may reach the optical system. Furthermore, by covering the main opening, the amount of light accessing the UCU and the optical system is reduced, which improves measurements by the optical system.

Figure 2 shows the profile view of a filter (214) (Figure 2A), a perspective view of the filter (Figure 2B), and a plan view showing the arrangement thereof in a funnel (21) (Figure 2C). It can be seen that the filter (214) has a hemispherical shape which helps to reduce splashes. However, in other embodiments, the filter (214) may have other shapes. For example, in another preferred form, the filter may have an essentially flat shape. It can also be seen that the filter has a series of openings (2141) which prevent the passage of large objects and urine bubbles and other liquids to the inside. The passage of any object with a width greater than the width of these grooves would be stopped. Since the filter is arranged over the main opening (212) of the funnel (21), the filter effectively filters the passage of unwanted elements to the main opening. However, in other embodiments, the filter (214) may not have these openings (2141), leaving a single space for the passage of the sample to the main opening (212) or the openings (2141) thereof may have other shapes that are even different from one another, where the entry of unwanted substances can thus be prevented.

Figure 2C shows the arrangement of the filter (214) in the funnel (21) in the central area thereof and covering a main opening (212). The filter (214) thereby prevents direct urination on the opening (212) and the entry of unwanted objects to same. However, in other embodiments, the main opening (212) may not be located in the central area of the funnel (21), in which case the filter (214) would not be located in said area either, but rather in the place where the main opening (212) were located. Furthermore, the filter (214) and/or the funnel (21) can in turn have different fixing mechanisms which allow the filter (214) to be fixed over the main opening (212) such that it does not move. The fixing mechanism may comprise one or more screws, nuts, openings, flaps, clips, grooves, friction systems, or any other element which allows fixing the filter (214) to the funnel (21) over the main opening (212).

Additionally, in a preferred embodiment, the filter may comprise a siphon mechanism configured to prevent gases from returning from the inner part of the funnel to the outside. The siphon mechanism may comprise different siphon systems. For example, the siphon may comprise interlinked elbows which generate a volume in a segment of the siphon that remains covered in water such that gases cannot run through the siphon. Alternatively, the siphon may comprise a membrane covering the passage of the siphon which yields to the pressure of the fluids running through the opening, but which is configured so that when there is no fluid running through the siphon, the membrane closes the siphon, preventing fluid communication of the inside of the funnel with the outside. In a preferred embodiment of the first aspect of the invention, the funnel further comprises a gasket in a part of the urinary measuring device which contacts the urinary receptacle for fluidically sealing a space between the urinary measuring device and the urinary receptacle. The gasket is therefore arranged at the height where the urinary measuring device is received by the inner walls of the urinary receptacle. In this sense, the location of the gasket is not limited by a specific height. However, for the purpose of preventing fluid accumulation, the gasket will preferably be arranged in the section of the funnel, more preferably in the upper part of the funnel. The gasket therefore provides the fluid it receives to the funnel, and the funnel in turn directs same towards the main opening thereof. Due to the sealing between the urinary receptacle and the gasket of the urinary measuring device, this gasket is fitted in the receptacle, preventing urine from running through anywhere other than through the urinary measuring device.

It should be highlighted that the gasket may have different shapes, including a circular shape, oval shape, or any other shape, including an irregular shape, in order to be adapted to the inner face of the urinary receptacle. Furthermore, the gasket can in turn be made up of different flanges, profiles, or protuberances which improve the fixing of the device to the urinal. Likewise, the gasket can in turn be made up of several gaskets arranged at different heights, providing an improved sealing level.

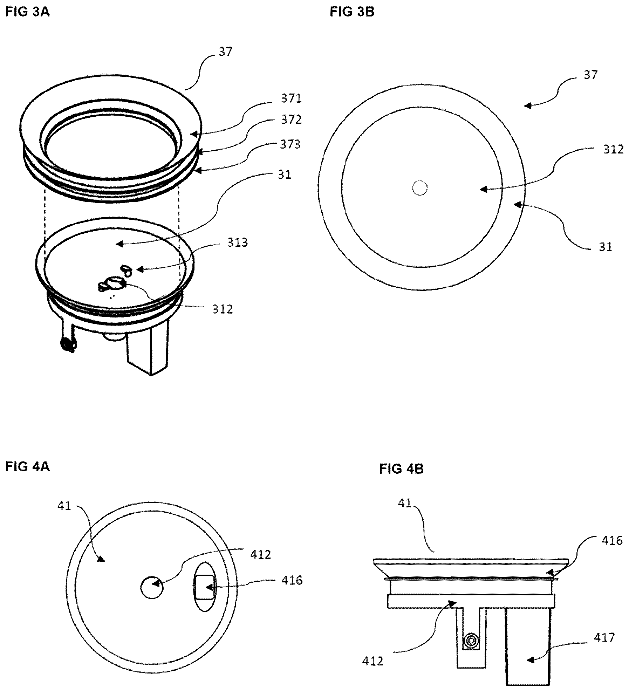

Figure 3 shows a gasket (37) in the arrangement thereof with respect to a funnel (31), both in an exploded view (Figure 3A) and in a plan view (Figure 3B). It can be seen that the gasket (37) has a circular design and is configured to be arranged on the outside of the funnel (31). However, in other embodiments, the gasket may have any other shape that allows the adaptation thereof to the funnel (31) and to the inner face of the urinary receptacle. Furthermore, the shape of the inner part of the gasket (37) which interacts with the funnel (31) can, in some embodiments, be different from the shape of the outer part of the gasket (37) which interacts with the urinary receptacle, where the shape of both does not have to be the same. The gasket therefore ensures leak-tightness of the urinary measuring device at the level of the funnel. Figure 3A shows fixing mechanisms (313) which allow a filter (not visible) to be fixed over the main opening (312) such that it does not move.

Furthermore, the gasket (37) comprises three sealing rings (371, 372, 373) at different levels, which ensure an improved fit with respect to the inside of the urinal and prevent urine from spilling out of the funnel (31). However, in other embodiments, the gasket (37) may comprise one or more gaskets a different levels or other mechanisms which ensures an improved fixing of the urinary measuring device to the urinary receptacle. Moreover, the gasket (37) is sealed to the funnel (31) by means of friction since the opening of the gasket (37) is smaller than the outer diameter of the funnel (31). Presumably, the gasket is preferably made up of a flexible material such that the sealing rings (371, 372, 373) push against the walls of the urinary receptacle when being inserted, so as to form a firm sealing. Furthermore, sealing is reinforced by the weight of the urinary measuring system, obtaining greater sealing when a sample is deposited on the urinary measuring device due to the weight of the sample. In other embodiments, the gasket and the funnel can interact by means of flanges, notches, or other fixing mechanisms other than the friction fixing mechanism. For example, in another preferred embodiment of the first aspect of the invention, the funnel (31) may comprise a screw mechanism which allows the coupling thereof to an external device comprising the gasket (37). In this sense, the funnel (31) can be coupled to a gasket (37) comprised as part of an adaptor element for a specific type of urinal, i.e., the gasket (37) may be part of a device with which the funnel (31) can interact through flanges, notches, or other fixing mechanisms other than the friction fixing mechanism.

In a preferred embodiment, the funnel further comprises an overflow opening configured to inhibit the sample from overflowing from the funnel, and a conduit configured to receive fluid from the overflow opening, with the conduit bypassing the UCU. After the discharge of water or cleaning fluid form a source of the urinary receptacle, the water flow received by the funnel increases considerably in comparison to a urination flow. Therefore, the main opening cannot account for the flow received and the water or fluid from the discharge is at risk of accumulating significantly on the funnel, thereby increasing the liquid elimination time during discharge. This overflow opening may also be incorporated directly in the toilet in a suitable place (and in this case, it would not be necessary to incorporate an overflow opening in the funnel). Although a gasket which seals the urinary measuring device may exist, it should be observed that the presence of fluids may cause damage to the different electrical and/or electronic part possibly comprised in the device. Therefore, the presence of an overflow opening in the funnel allows mitigating these problems. Its size is not limited, although it is preferably large enough so as to, along with the main opening, deal with the water flow coming in from the tank.

It should be highlighted that the overflow opening is preferably arranged in a more elevated position than the main opening. By being at a greater height, it is achieved that most, if not all, of the sample is collected during urination through the main opening which suitably directs same to the UCU for analysis. Furthermore, when the discharge of water or liquid occurs, the level of the liquid rises (as a result of a larger inflow than outflow in the funnel), and only then the overflow opening is used to evacuate water and thereby accelerate the elimination of water from the urinary measuring system. However, in other embodiments, the overflow opening may not be located at a height greater than the height of the main opening, and other mechanisms may be used to receive water in the case of a risk of overflow.

Moreover, it should be highlighted that the overflow opening provides the excess fluid through a conduit that serves to prevent exclusive collection by the UCU. Part of the deposited sample being redirected by the overflow opening has the advantage of accelerating the water elimination time of the urinary measuring system, in addition to reducing the exposure and use of the optical measuring system given the need to process less volume during discharges, thereby prolonging the service life thereof. However, there will always be a part of water coming from the part that goes through the main opening and is provided to the UCU, allowing the UCU to be cleaned, the systems to be calibrated, and the condition of the device to be understood (whether it contains urine or water, among other derived calculations).

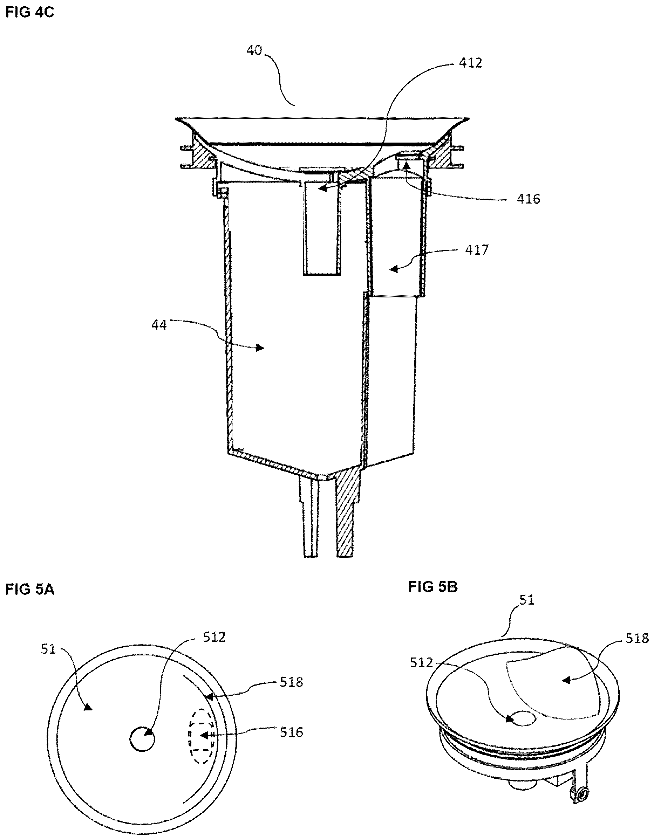

Figure 4 shows a funnel (41) with an overflow opening (416) in a plan view (Figure 4A), a profile view (Figure 4B), and a profile view with a cross-section (Figure 4C). It can be seen in Figure 4A how the overflow opening (416) is arranged on one side of the funnel (41). The overflow opening (416) allows, in the case of overfilling the funnel (41), the possibility of passively relieving the amount of water stored therein. Since it is an overflow opening, when the level of the sample reaches the height of said opening, it is discharged through the overflow opening (416). However, in other embodiments, the overflow opening (416) can be placed in other locations which are not on a side.

Furthermore, Figure 4B shows that the overflow opening (416) is arranged at a height greater than the height of the main opening (412). This allows the sample to be directed through the main opening (412) in urination situations, whereas the overflow opening (416) helps the evacuation thereof in water discharge situations. In other embodiments, the main opening (412) and the overflow opening (416) can be placed at any point of the funnel (41), and therefore the overflow opening (416) may not be located at a height greater than the height of the main opening (412).

Lastly, it can be seen in Figure 4C how in the urinary measuring device (40), unlike the main opening (412), the overflow opening (416) provides fluids through a conduit (417) such that they are not measured. In some embodiments, the conduit (417) may not be present and/or may have other shapes and/or may redirect the fluids such that they were indeed measured by any other measuring system.

Optionally, in a preferred embodiment, the funnel further comprises a lid on the overflow opening configured to prevent the sample from flowing directly through the overflow opening. Despite the overflow opening preferably being arranged in a position which reduces the possibility of the user urinating directly, there is the possibility of the user urinating on the overflow opening, which would entail a partial or complete loss of the sample. By means of this lid, direct access to the overflow opening is not possible directly with a reasonable margin from the directions in which urination may take place, so as to cover the different positions that the user may accidentally acquire. Generally, these positions are vertical and front positions, although depending on the urinary receptacle and on the position of the overflow opening, other positions may be possible, and are therefore object of the present invention. However, the lid allows indirect access of the sample to the overflow opening, in the case where overflow is a possibility. In some embodiments, this means that horizontal access to the overflow opening is allowed, although this restriction may take different considerations according to the embodiment. Therefore, the lid does not prevent the evacuation of water when the fluid level in the funnel reaches the level of the overflow opening.

It should be highlighted that the shape of the lid is not limited in terms of size or form, provided that it prevents vertical and/or direct access of the fluid to the overflow opening, and allows horizontal access, i.e., access as a result of an overflow. Therefore, the lid can be curved to prevent splashes and/or with an angle that directs the fluid to the funnel or main opening or prevents it from being directed out of the funnel.

Figure 5 shows a funnel (51) in a plan view (Figure 5A) and in an isometric view (Figure 5B), comprising a main opening (512) and a lid (518) covering the overflow opening (516). In Figure 5B, it can be seen how the lid (518) is arranged such that it prevents direct urination on the overflow opening (516) (not visible), but still allows horizontal access thereto, allowing the access of the sample in the case of an overflow. Furthermore, the lid (518) tapers towards the main opening and has rounded shapes to reduce splashes. In this way, the lid (518) adopts the shape of a flap on the overflow opening (516). However, in other embodiments, the lid (518) can adopt other shapes which are not rounded or which preferably do not direct the fluids to the main opening (512). Furthermore, in other embodiments, the lid (518) and the filter of the main opening (512) can be attached or can work together in order to preferably provide the fluids to the main opening. Furthermore, in other embodiments, the lid (518) can be a separate element of the funnel (51) or can be provided from another element of the urinary measuring device, such as a gasket.

In a preferred embodiment of the first aspect of the invention, the light emitter and the light sensor are located in the casing for electronics for emitting and receiving light with respect to the UCU such that the planes perpendicular to the emission and measurement directions form an angle comprised between 120° and 180°. If the emitted light and the measured light have several directions, the central directions are taken. The optical measurement which allows taking several measurements of the sample involves interaction of the light emitted by the light emitter, the urine sample placed in the UCU, and the light sensor. The light emitted by the light emitter interacts with the sample, and the light sensor measures the light resulting from that interaction. In the cases where spectrophotometry is performed on the sample by means of incident light refraction and reflection, the position of these three elements is purposely pre-arranged at preferred angle and distances following Snell's law of electromagnetism. It follows, therefore, that the sample must therefore at least partially cover the UCU for spectrophotometry to be carried out.

Snell's law provides that the multiplication of the refractive index by the sine of the angle of incidence with respect to the normal is constant for any ray of incident light on the interphase between two different transmission media. Therefore:

$$n_1 sin(\theta_1) = n_2 sin(\theta_2)$$

for two media 1 and 2 with refractive indices $n_1$ and $n_2$, respectively. It is therefore possible to calculate the path of an incident light beam on a medium, as well as the exit thereof after being reflected at the bottom. Therefore, it is possible to calculate the angle at which the light emitter and the light sensor are arranged, the light sensor being capable of measuring the light reflected by the UCU containing the sample. Since the light emitter and/or the light sensor can be configured to emit/receive light over a range of angles, and the surface of the UCU can adopt a curved or concave shape, it can be seen that the light emitter and the light sensor can be oriented in a range of configurations and still be capable of detecting light which has passed through the sample.

Furthermore, it should be highlighted that the optical system is preferably arranged a distance from the UCU, such that the amount of splashes on the optical system is reduced, allowing a more durable system than if it were submerged or in direct contact, since deterioration of the optics due to lime and dirt is prevented.

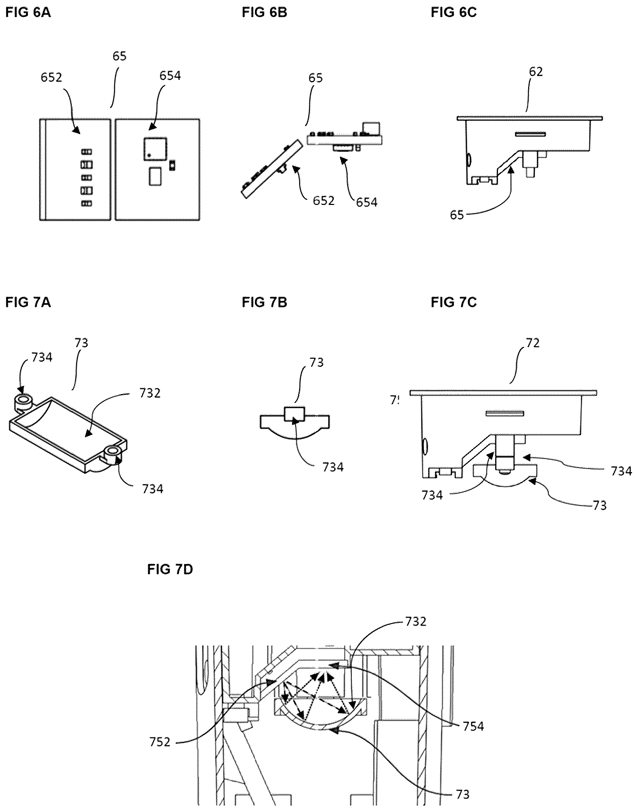

Figure 6 shows an optical system (65) in a plan view (Figure 6A) and a profile view (Figure 6B) and a casing for electronics (62) housing the optical system (65). It can be seen in Figure 6A that the optical system comprises a light emitter (652) which in turn comprises several LED diodes which allow emitting light to perform a spectrophotometry test. In other embodiments, the light emitter (652) may comprise other light emitters in different combinations. Similarly, it can be seen that the optical system (65) comprises a light sensor (654) which in turn comprises several sensors which allow detecting light coming from a sample in a plurality of wavelengths over a spectrum to be analyzed, after having interacted with the sample to perform a spectrophotometry test. In other embodiments, the light sensor (654) may comprise one or more sensors of different types (different methods for measurement, sensitivity to different frequencies). It can be seen in Figures 6B and 6C that the light emitter (652) and the light sensor (654) are arranged at a predetermined angle. In other embodiments, the predetermined angle can be different. Furthermore, it can be seen particularly in Figure 6C that the casing for electronics (62) containing the optical system (65) is configured to arrange the light emitter (652) and the light sensor (654) at the desired predetermined angle. Although not present in this embodiment, the optical system (65) of other embodiments may also comprise a protector which protects the exposed face of the optical system against the sample splashing from the UCU.

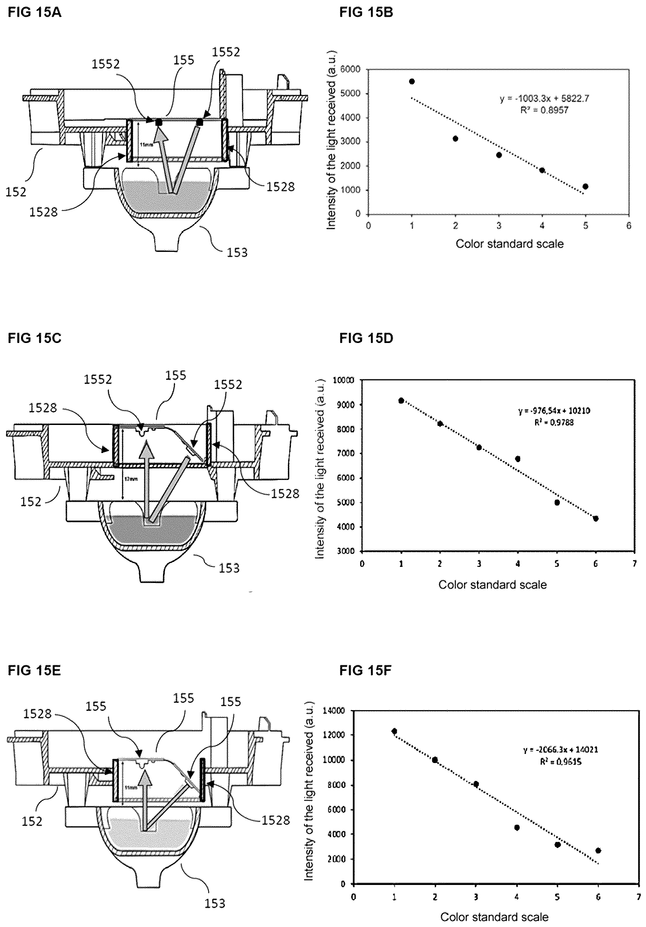

In another preferred alternative embodiment of the first aspect of the invention as shown in Figure 15A, the light emitter (1552) and the light sensor (1554) of the optical sensor (155) are located in the casing for electronics in order to emit and receive light with respect to the UCU (153) such that the planes perpendicular to the emission and measurement directions form an angle of preferably 180°, that is, with the same direction and sense. If the emitted light and the measured light have several directions, the central directions are taken. The optical measurement which allows taking several measurements of the sample involves interaction of the light emitted by the light emitter (1552), the urine sample placed in the UCU (153), and the light sensor (1554). The light emitted by the light emitter (1552) interacts with the sample, and the light sensor (1554) measures the light resulting from that interaction. In the cases where spectrophotometry is performed on the sample by means of incident light refraction and reflection, the position of these three elements is purposely pre-arranged at preferred angle and distances following Snell's law of electromagnetism as described above.

Figure 15 shows several alternatives of a casing for electronics (152) housing the optical system (155) and an UCU (153). It can be seen that the optical system comprises a light emitter (1552) which in turn comprises several LED diodes which allow emitting light to perform a spectrophotometry test. In other embodiments, the light sensor (1554) may comprise one or more sensors of different types (different methods for measurement, sensitivity to different frequencies). It can be seen in Figures 15C and 15E that the light emitter (1552) and the light sensor (1554) are arranged at a predetermined angle. In other embodiments, as shown in Figure 15A, the predetermined angle can be different, for example, with light being provided to perform a spectrophotometry test forming an angle of 180°. In this sense, the present embodiment is not limited to a specific type of angle between the light emitter (1552) and the light sensor (1554).

Furthermore, it can be seen in Figures 15C and 15E that the optical system (155) is arranged at different distances from the UCU (153). In other embodiments, the optical system (155) can be arranged in direct contact with the UCU (153), at a distance far away from the UCU (153) as shown in Figure 15C, or at a distance close to the UCU (153) as shown in Figure 15E. Advantageously, this allows different emitter and receiver technologies (different methods for measurement, sensitivity to different frequencies) which require and/or allow the use of different distances between the optical system (155) and the UCU (153) to be used in the light device to perform a spectrophotometry test. In this sense, the present embodiment is not limited to a distance between the light emitter (1552) and the light sensor (1554).

Furthermore, it is observed that the optical system (155) is comprised between optional walls (1528) of the casing for electronics (152) configured to prevent light from scattering in unwanted directions. Advantageously, this allows directing and maximizing the projection and reception of light between the light emitter (1552) and the light sensor (1554). Preferably, the walls (1528) define a surface in the direction of measurement by the optical system (155) to and from the UCU (153).

Figures 15B, 15D, and 15F show the different standard curves of the optical systems (155) of Figures 15A, 15C, and 15E, respectively, in comparison with a color standard. The color standard is made up of a color scale where the value 0 represents transparency and the value 6 represents the most intense color which therefore absorbs a higher amount of light. Therefore, a larger number represents a more concentrated urine. As can be seen, in all the cases, regardless of the angle at which the light emitter (1552) and the light sensor (1554) are arranged and of the distance between the optical systems (155) and the UCU (153), the light sensor (1552) is capable of measuring the light received from the UCU (153) with great precision in all the cases, such that the standard curve has an $R^2$ fitting of at least 0.89.

In a preferred embodiment, the casing for electronics comprises a main body, a cover, and a gasket configured to fluidically seal the interface between the main body and the cover. The gasket allows fluidically sealing the main body and the cover, preventing the entry of fluids into same in the event that a fluid comes into contact with the casing for electronics. This therefore provides greater durability to the electronic system.

In a preferred embodiment, the casing for electronics comprises a transparent protector arranged for protecting the light emitter and/or the light sensor against the sample splashing from the UCU. Since the light emitter and the light sensor are directed towards the UCU, it is possible that certain splashes of the sample may reach these sensors from the UCU. Therefore, the transparent protector allows protecting the light emitter and the light sensor from splashes, prolonging their service life and preventing erroneous readings, as would occur if a drop of sample were to partially or completely block the light emitter or the light sensor.

The transparent protector can be formed from any material which allows the light to be transmitted to the UCU from the light emitter and allows the light reflected from the UCU to reach the light sensor, including any of the following: styrene methacrylate (SMMA), polycarbonate, poly(methyl methacrylate) (PMMA), quartz, amorphous quartz, glass, chemically reinforced glass (such as Gorilla Glass or Xensation Schott glass), or sapphire crystal. The exposed face of the transparent protector can be covered with a coating to prevent the formation of bacteria (for example, an anti-bacterial coating) and/or to prevent the degradation of the protector as a result of the splashes.

In a preferred embodiment, the UCU comprises a surface arranged for maximizing the reflection of an incident light and which in turn comprises a material selected from one of the materials from the following list: ceramic, crystal, glass, porcelain, aluminum, polymers such as POM or a derivative of these materials. The surface of the UCU can also be treated with different agents and/or processes such as, but not limited to, specific coatings or paints causing the surface to have optimal whiteness to perform the analysis. The selection of these materials allows an UCU that is durable in the face of various aggressive agents (lime, urea, detergents, etc.) and facilitates the directing of the light coming from the light emitter to the light sensor. Furthermore, the shape of the UCU may favor the reflective effect of these materials, such that incident light is directed more directly to the light sensor.

Figure 7 shows a urine collection unit (UCU) (73) in a perspective view (Figure 7A) and in a profile view (Figure 7B), a casing for electronics (72) housing an optical system (75) and with the UCU (73) being arranged in the central area thereof, where a funnel (not visible) would deposit the sample (Figure 7C), and a profile view (Figure 7D) of the interaction of the light on the surface of the UCU (732). It can be seen in Figures 7A and 7B that the UCU (73) has a concave inner surface (732), which allows increasing the amount of light reaching the light sensor, increasing the sensitivity of the system. Other embodiments may not have this surface or may have a surface with another shape different from that of this embodiment. The UCU (73) comprises one or more fixing elements (734) which allow fixing the UCU (73) to other components of the apparatus. In the illustrated embodiment, the fixing elements (734) allow the fixing thereof to the casing for electronics, whereas in other embodiments, the fixing elements (734) may allow the fixing thereof to other elements of the apparatus, such as the funnel. These fixing elements may comprise one or more screws, nuts, openings, flaps, clips, grooves, friction systems, or any other element which allows fixing the UCU to another element of the urinary measuring device.

Furthermore, it is seen in Figure 7C that the UCU is positioned a certain distance from the optical system (75) contained in the casing for electronics (72), which prevents splashes and deterioration as a result of lime and the formation of films on the lenses. In other embodiments, the UCU may be positioned in contact with the optical measuring system. It can also be noted how the optical system (75) establishes a different angle with the UCU (73) for the light emitter and for the light sensor such that the light emitted by the light emitter is reflected on the sample contained in the UCU and received by the light sensor. It can also be seen how the fixing element (734) allows the UCU to be fixed to the casing for electronics in this particular figure. Although not present in this example, in other embodiments, the casing for electronics (72) may comprise a transparent protector protecting it against the sample splashing from the UCU.

It is shown in Figure 7D how the UCU (73) is positioned and configured to converge all the light beams coming from the light emitter (752) in the light of the light sensor (754). In this preferred embodiment, the surface of the UCU is in the form of a reflective lens and the light sensor is positioned close to or at the focus point, in order to maximize sensitivity. This improves light detection by the light sensor. In other embodiments in which light reflection is used to receive light in the light sensor, the position and shape of the UCU may or may not achieve the convergence of light beams in the light sensor through other means, as long as part of the reflected light reaches the light sensor. The importance of the UCU having a surface (732) that reflects light sufficiently is also observed.

In a preferred embodiment, the UCU comprises a series of side recesses (not shown) which allow establishing a maximum urine level in the UCU that is lower than the maximum height thereof, such that the UCU is not filled up completely, but rather there is always an upper section of the UCU that has no urine. Advantageously, this allows the urine level to always be the same level, facilitating homogenous measurements by the optical system, while at the same time eliminating urine from the UCU in a controlled manner.

In an even more preferred embodiment, the UCU comprises an outer surface in the lower part of said side recesses which allow controlling the running of the urine and fluids on the UCU during the outflow thereof. The UCU preferably comprises an outer surface the base of which comprises a rounded shape with a truncated cylinder at the end thereof, such that the urine and fluids exiting the UCU through the recesses (not shown) run along the outside of the UCU towards said truncated cylinder, where they are finally eliminated from the UCU to subsequent stages of the urinal. Advantageously, this allows the flow from the UCU to the subsequent stages of the urinal to be carried out in a controlled manner.

In a preferred embodiment, the urinary measuring device further comprises:

 d. A container for collecting the fluid overflowing from the UCU, comprising an outlet opening; and

 e. A plurality of electrodes extending inside the container by means of arms.

The device may comprise a voltage source connected between the electrodes configured to apply a voltage signal between the electrodes and a current meter configured to measure the current between the electrodes resulting from a voltage signal applied by the voltage source. The device may comprise an impedance meter connected to the electrodes, the impedance meter configured to measure the impedance of the fluid present in the container between the electrodes.

In this preferred embodiment, the container is arranged such that the fluid overflowing from the UCU is collected by the container. Therefore, the container is capable of housing a fluid. Moreover, the outlet opening is arranged so as to allow the exit of the collected fluid, preferably in the lower part of the funnel, such that the sample contained in the container can be evacuated entirely through the outlet opening. The electrodes can be arranged in any part of the container where the sample will be collected, preferably in a position close to the position of the outlet opening. In some embodiments, the electrodes can be arranged by means of a plurality of arms extending from another element of the device, such as the casing for electronics. In a preferred embodiment, the outlet opening of the container is configured to allow a predetermined outflow that is smaller than that of the main opening of the funnel. By means of the design of the outlet opening of the container, the flow of the fluid running through the container can be controlled. By having a flow that is smaller than that of the main opening of the funnel, there is also an opening with a flow smaller than that of the inflow of the funnel from the UCU as a result of an overflow. Therefore, when receiving a fluid from the UCU, an effect of filling the container with the incoming fluid, either the urine sample or water from the water tank, occurs.

The advantage of knowing the size of an outlet opening is that it can be used to calculate the total volume of fluid running through the opening. If the speed of the fluid through said opening is known and the time (t) it takes for all the fluid to run is calculated, the total volume of fluid which has run through the opening can be obtained. In this sense, it is possible to predict the speed of a fluid (v) running through an opening for a certain fluid column having height (h) by means of Torricelli's law defined as:

$$v = \sqrt{2gh}$$

where g is the universal gravitational constant. One of the conclusions of this equation is that the flow changes with the height of the fluid column. Therefore, it is necessary to take additional considerations for obtaining measurements which take into account this change in height.

If certain assumptions are made, such as the opening being arranged in the lower part of a cylinder with a base having an area A, where the opening covers an area a, and a height h, the outflow speed v can be determined as:

$$v = \frac{dx}{dt} = \sqrt{2gh}$$

where dx is the equivalent height of a fluid cylinder having an area a formed by the fluid contained in the container having an area A. Therefore, the following can be defined:

$$dx = \frac{A}{a}\, dh, \qquad from\ which\ it\ follows\ that\ \frac{dx}{dt} = \frac{A\,dh}{a\,dt} = \sqrt{2gh}$$

By solving and integrating, the time elapsed between an initial height $h_i$ and a final height $h_f$ is obtained as follows:

$$t = \frac{2A}{a\sqrt{2g}}\left(\sqrt{h_i} - \sqrt{h_f}\right)$$

and given that the change in height of the water column is related to the volume V as follows:

$$\Delta h = \frac{\Delta V}{h}$$

it follows that:

$$\Delta t = \frac{2A}{a\sqrt{2g}}\sqrt{\frac{\Delta V}{h}}$$

Therefore, it is possible to establish a relationship between the volume of the liquid stored in the cylindrical container having an area A and the time elapsed in emptying a fluid column having a height h through an opening having an area a.

It is possible to establish this relationship for other shapes of the container, but additional steps may be required. For example, although linking the volume with height is more complex for a bowl-shaped container in which the outlet opening is in the center, in practice this shape provides a volume-time relationship close to linearity. This is due to the fact that, although the pressure exerted by the fluid column is lower when emptying the container, the bowl shape maintains a constant pressure in the outlet opening. It is even possible to define container shapes which ensure a constant outflow, like in the case of outflow water clock.

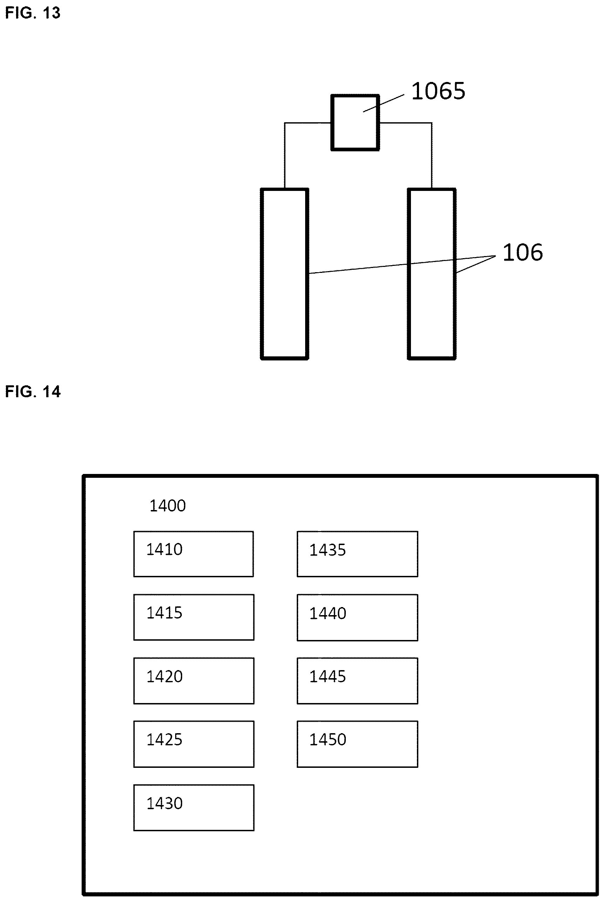

Therefore, it follows that, given a container with a defined shape, it is possible to determine the volume flowing through an opening made in the container, given a known water height and the time taken for emptying same. In the case of a design which ensures a constant flow, the height would be negligible. It should also be highlighted that, for a container with an arbitrary shape, the relationship between the time it takes to empty the container and the total volume can be determined empirically through observations which link the time elapsed with the volume of liquid which has exited the container and stored in a lookup table. The system can then compare the time elapsed in the lookup table and determine the total volume. Alternatively, the total volume can be determined through predetermined equations and algorithms taking into account a known volume, the flow rate, the outlet opening, and the emptying time. In this manner, the urinary measuring system can then correlate the time elapsed through the algorithms saved in a memory (for example, in a memory of the device or in an external memory such in the cloud) in order to determine the total volume. Alternatively, a machine learning algorithm which is trained can be used to link total urination time with total urination volume for a container with a specific shape (the machine learning algorithm can be trained using a training data set comprising the time it takes to empty the specific container for a plurality of known fluid volumes). Once the total volume and the total urination time are calculated, the urination flow rate can also be calculated as wall. In the case of hydration, the present system determines the specific level of hydration through electrical impedance as indicated in relation to Figure 14.

In a preferred embodiment in which the container has no walls with regular shapes or in which the shape of the container hinders the measurement of the total volume, the container comprises in its base longitudinal walls around the outlet opening, configured to generate a regular volume in the form of the container around the outlet opening and in which the plurality of electrodes can be placed. Advantageously, this allows the fluid to flow through the outlet opening from said volume which, due to the longitudinal walls, allows a more precise and regular measurement of the total volume by means of the plurality of electrodes. More preferably, the longitudinal walls define a cylinder.

In the embodiments in which the UCU comprises a series of side recesses and an outer surface in the lower part of said side recesses, which allow controlling the running of the urine and fluids on the UCU during the outflow thereof, the outer surface of the UCU directs the urine flow from the UCU to the container defined by the longitudinal walls of the container. Even more preferably, the UCU directs the urine flow from the UCU to the container through a rounded shape with a truncated cylinder at the end thereof, such that the urine and fluids exiting the UCU through the recesses (not shown) run along the outside of the UCU to said truncated cylinder, where they are finally directed to the container.

The filling of the container allows the electrodes arranged therein to come into contact with the fluid. This in turn allows taking, by means of the electrodes, additional measurements of the fluid, either urine sample or water, related to its electrochemistry. In some embodiments, the urinary measuring device further comprises an impedance meter connected between the electrodes which allows measuring the impedance of the medium between the electrodes. The impedance meter can be any type of impedance meter for measuring the impedance between the electrodes. The impedance meter can transmit a voltage signal (which can be an AC or DC signal, preferably an AC signal) through one of the electrodes and measures the impedance profile of the medium through the reception of the same signal by the other electrode. The signals may have a frequency preferably between 1 and 10M Hz or 1 and 100 MHz, preferably sinusoidal frequencies.

In some embodiments, the impedance meter measures the complex impedance between the electrodes. Complex impedance can be measured by calculating the relationship between the complex representation of the sinusoidal voltage signal through the electrodes, and the complex representation of the current flowing through the electrodes. Any type of complex impedance meter can be used.

Figure 13 shows the arrangement of an impedance meter according to one or more embodiments. The impedance meter 1065 is arranged between the electrodes to measure the impedance of the medium arranged between the electrodes 106. In other embodiments, several electrodes can be connected to several impedance meters and/or several impedances can share one and the same electrode by way of reference.

The electrodes are preferably monitoring the impedance of the medium either intermittently or continuously. When the impedance of the medium in which the electrodes are located is greater than a threshold, i.e., when the impedance thereof drops below a certain value, a sample can be defined as having come into contact with the electrodes, and therefore as being present. Likewise, it is determined that a sample is not present when the impedance between the electrodes is above a certain threshold. This allows making decisions depending on whether or not a sample is present.

Furthermore, a series of electrodes can be arranged at different heights, where the device comprises one or more impedance meters configured to measure the impedance between the electrodes at a specific height. This allows precisely detecting the sample level in the container, as different electrodes report different impedances. Furthermore, it allows knowing the estimated container emptying time before the container finishes emptying, or if the container is filled in midway through the process, detecting it. The various electrodes can be arranged in the same arm or in different arms. One electrode can serve as a reference electrode common to several measurement electrodes.

In a preferred embodiment, the container has a cylindrical shape and is configured to provide disponer a fluid through its outlet opening at a constant speed.

In a preferred embodiment, the container further comprises a side outlet opening, arranged at a greater height than that of the outlet opening and than that of the plurality of electrodes. Given that, in some embodiments, the outlet opening of the container may allow a flow lower than the inflow, an evacuation system which prevents excessive filling of the funnel can be provided, affecting the optical system if it is arranged inside the container. This effect is achieved by means of one or more outlet openings on the side of the container. Furthermore, the height of these side openings is selected such that it is lower than the height of the optical system, but higher than the location of the electrodes. In this way, the side openings do not interfere with the measurement function of the electrodes and protect the electronics from excessive exposure to the fluid.

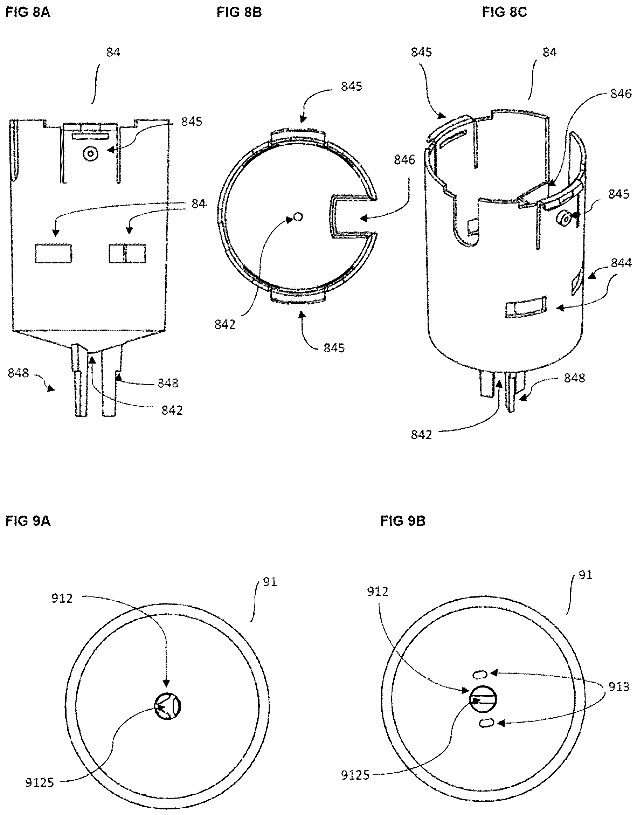

Figure 8 shows a container (84) in an elevational view (Figure 8A), in a plan view (Figure 8B), and in a perspective view (Figure 8C). It can be seen in Figure 8A that the container (84) has two side outlet openings (844) arranged at a mid-height of the container. These two openings allow the sample contained therein to be evacuated in the case where the level is high, such that the sample contained therein is prevented from reaching the electronic and/or measuring systems. The height of these openings is limited by the casing for electronics, the optical system, and the UCU, since the container (84) is designed for housing all these elements; and it is always located at a height greater than the height of the outlet opening (842). However, in other embodiments, the container (84) may not have these side openings (844) or may have only one or more than two side openings (844). Furthermore, the height and design of the openings may vary according to the embodiment, which may not house any of the elements such as the casing for electronics, the optical system, and/or the UCU.

It should also be noted that the base of the container (84) has a conical shape in the form of a funnel which allows it to direct fluids to the outlet opening (842). Other embodiments may not have of any shape in the base of the container (84) which will help them to direct fluids. Likewise, the outlet opening (842) may not be arranged in the center of the base of the container (84), or even in the lowest part thereof. In the container (84), a series of flanges (848), each of them comprising a support surface (8482), help to securely fix the container (84) to a urinary receptacle by means of the interaction of the support surfaces (8482) with a lower surface of the urinal. In other embodiments, the container (84) may have another type of systems (848) which help to fix or support same on the urinary receptacle, or it may dispense with said systems. If the urinary receptacle is a toilet, the anchoring systems may comprise flanges configured to secure the device to the edge of the toilet or any other system which allows fixing the device in the toilet.

Figure 8B shows the plan view of the container (84) having the outlet opening (842) in the center thereof. However, in other embodiments, the outlet opening (842) may not be arranged in the center of the base of the container (84). Furthermore, the presence of a recess (846) extending along the entire height of the container (84), which is configured to accommodate the conduit (417) conducting the fluids coming from the side opening of a funnel bypassing the UCU and the container, should be highlighted.

It can be seen in Figure 8C that the container (84) also has a series of fixing elements (845) which allows the fixing thereof to other elements such as, for example, a funnel. These fixing elements may comprise one or more screws, nuts, openings, flaps, clips, grooves, friction systems, or any other element which allows fixing the container (84) to another element of the urinary measuring device. The fixing elements (845) can be compressed to release the flanges of the casing for electronics. The recess (846) which allows accommodating the conduit that allows the sample to bypass the UCU and the container (84) can also be seen.

In a preferred embodiment, electric cables extend from the arms to the casing for electronics through a gasket, in order to form an electrical connection between the electrodes and the electronics in the casing for electronics.

In a preferred embodiment, the funnel comprises a handle configured to facilitate the extraction of the urinary measuring device by means of an extraction device. The handle is arranged on the upper surface of the funnel so that it is accessible when the device is installed in the urinal or toilet. For the extraction of the urinary measuring device, various extraction devices with hooks can be used, such that the operator removing the urinary measuring device does not come into direct contact with the device, minimizing the risk of exposure to pathogens. To that end, a handle arranged in the funnel allows securely grabbing the urinary measuring device with the extraction device, overcoming the weight thereof and the resistance caused by the friction of the gasket with the inner surface of the urinary receptacle. Preferably, the handle is arranged on the main opening of the funnel, but it can be located in any part of the funnel which is accessible from the upper surface of the funnel.

Figure 9 shows two embodiments (Figure 9A and Figure 9B) of a funnel (91) in a plan view, in which the main opening (912) has a handle (9125) which allows extracting the urinary measuring device by means of an extraction device. It is observed that the handle (9125) is a representative mode and can adopt different shapes in other embodiments. Therefore, in Figure 9A, the handle has a triangular shape, whereas in Figure 9B, it has a rectangular shape. For example, the handle can adopt bulk shapes projecting from the plane of the surface of the funnel. Likewise, the handle (9125) can be located in different location in the funnel (91), where the location thereof is not limited to the main opening (912). Therefore, the handle (9125) can be comprised in any location in the funnel (91). In some embodiments, it is also possible for the handle (9125) to be made up completely of another element of the funnel (91), such as a covering or the main opening (912) itself. Lastly, Figure 9B shows the funnel (91) having two fixing mechanisms (913) which allow fixing a filter (not visible) over the main opening (912) to prevent direct urination and the entry of objects and urine foam through the main opening.

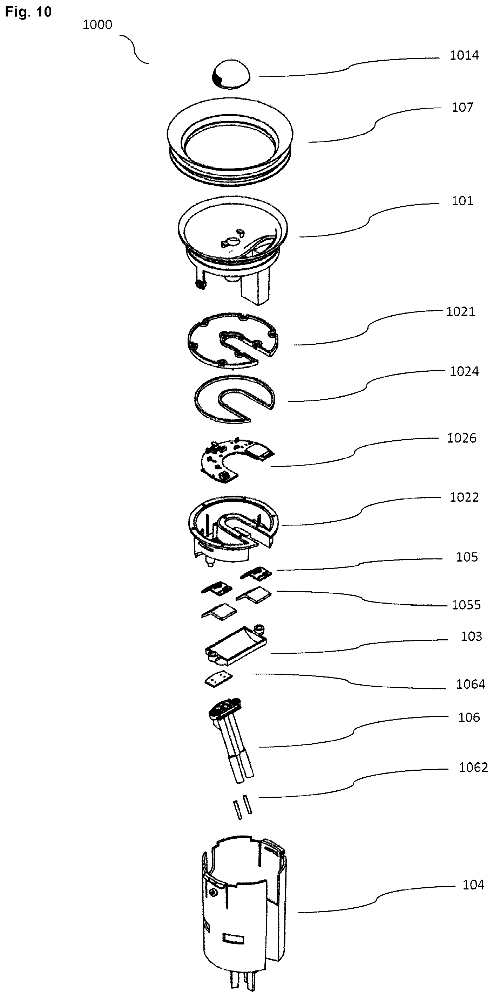

Figure 10 shows an exploded view of a urinary measuring device (1000) according to an embodiment. The urinary measuring device (1000) has a filter (1014) over the main opening of the funnel (101) which prevent the entry of substances which may damage the device through the main opening. In some embodiments, this filter is not provided. The filter can be like the one described in reference to Figures 2A - 2C.

The device also has a gasket (107) in the outer part of the funnel configured to prevent the spillage of samples between the funnel (101) and the urinary receptacle. This gasket can be like the one described in reference to Figures 3A and 3B. Some embodiments are not provided with the gasket (107) of the funnel (101).

Furthermore, it is observed that the device has a casing for electronics made up of an upper cover (1021), an isolating gasket (1024), and a base (1022). This casing for electronics can be like the one described in reference to Figure 6C. In some embodiments, the casing for electronics is not provided with the isolating gasket and/or is formed by more or less pieces. The casing for electronics houses in the inside thereof a controller (1026), although not limited thereto, and in the lower part thereof an optical system (105). Some embodiments do not have a controller (1026). For example, the casing for electronics can be configured to transmit and receive control signals and/or to receive sensor data from the light sensor and/or the impedance meter in order to control the electronic components of the device through an external controller (for example, by means of a receiver or by cabled connections to the external controller). The optical measuring system has protectors (1055) which protect the optical measuring system from possible splashes. These protectors are optional in some embodiments.

The urinary measuring device (1000) also comprises a urine collection unit (UCU) (103) fixed to the casing for electronics through bolts. This urine collection unit can be like the one described in reference to Figures 7A-7D. The UCU (103) allows collecting samples coming from the main opening of the funnel (101). In some embodiments, the UCU (103) can be fixed to other elements of the device and by other means, such as those described above.

Moreover, the device comprises two electrodes (1062) contained in one arm (106) attaching same to the casing for electronics. The arm (106) allows the electrodes (1062) to be arranged in a preferred position, and the electrodes (1062) allow the signal circulating between both to be measured. Although this embodiment shows two arms (106), other embodiments may have only one arm, more arms, or may not have any arms at all. Furthermore, the electrodes (1062) of the arm (106) can be arranged at different heights and have different shapes according to the embodiment. In this embodiment, the arms (106) comprise a gasket in the attachment thereof with the casing for electronics which prevents the entry of fluids therein. In other embodiments, the gasket (1064) is not provided.

Lastly, the urinary measuring device (1000) has a container (104) collecting samples that overflow after the filling of the UCU (103) and arrange it such that the electrodes (1062) can be submerged when a sufficient amount of sample is introduced. In some embodiments, the container (104) is not provided. The container can be like the one described in reference to Figures 8A-8C.

In a preferred embodiment, the casing for electronics further comprises a controller configured to control the light emitter, the light sensor, and optionally the impedance meter, and wherein the controller is made up of a processor configured to process information from the light sensor and the impedance meter. The casing for electronics can contain an internal energy source such as a battery, or can be connected to an external energy source, and the controller selectively directs the energy to the different components. Alternatively, the casing for electronics comprises a communication interface for wired or wireless communication with an external processor and/or a controller which are configured to process and/or control information from the optical system and/or the electrodes.

The controller is configured to control the optical system by means of an interface (the light emitter and the light sensor) and the electrodes, and to receive information from the optical system and the electrodes and to process said information. Therefore, the processor is configured to control when to activate the light emitter, when the light sensor detects the light it receives, and when to activate the impedance meter (if measurement is taken intermittently). Similarly, the processor receives information about the light detected by the light sensor and about the impedance of the medium. Lastly, the processor is configured to process the information received in different ways: it calculates parameters of the information obtained and sends signals to the components by means of the interface, turning the components on and off.

Optionally, the controller may comprise a memory. The memory is configured to store the instructions executed by the processor, as well as to store information received by the processor and information processed by the processor. Furthermore, the processor can be configured to be connected to a computer external to the urinary measuring device, such as a cloud server, in order to transmit and receive information.

All these connections can be made by electric or electronic means through cables of any type and/or as telecommunication protocols such as WiFi, Bluetooth, NFC, among others.

It should be highlighted that the processor may comprise one or more processing units, such as a microprocessor, a GPU, a CPU, a multicore processor, or the like. Similarly, the memory may comprise one or more volatile or non-volatile memory devices, such as DRAM, SRAM, flash memory, read-only memory, ferroelectric RAM, hard disk drives, floppy disks, magnetic tapes, optical disks, or the like. The controller can be implemented in software (a computer program), hardware (a physical device), or any combination for the purpose of executing the operation sequences disclosed herein.

In a preferred embodiment of the first aspect of the invention, the urinary measuring device further comprises a valve of the urinary receptacle configured to control the outflow of water from a water source to the urinary receptacle, wherein the device is configured to control the valve. The valve can be arranged both inside a tank in an upper wall of a urinal and in the shutoff cock for shutting off the water feeding the tank, so as to have control over when the water from the tank is deposited in the urinary receptacle. In this embodiment, the processor of the controller is preferably configured to control the valve, so it can control when to open and close the valve. Similarly, if the controller comprises a memory, the memory is preferably configured to store the instructions of the processor which allow it to control the valve. It should be highlighted that the valve can be of different types, such as solenoid valves or pneumatic control valves. In the last case, the device also comprises a pneumatic pump system which allows controlling the pneumatic control valve. In some embodiments, the urinary measuring device is configured to open the valve when it detects that sample measurement has ended.

In another preferred embodiment, the urinary measuring device further comprises a user interface configured to receive identification or anonymous information of a user (i.e., information that does not identify the user) and to show information about the condition of the sample to the user. The user interface can therefore receive a user input which identifies the user and provide said user with information of interest throughout the urination process, analysis, or at the end of the process. In this embodiment, the processor of the controller is preferably configured to receive and transmit information of the user interface, so it can receive information about the identity of the subject and transmit information about the urine analysis performed, as well as the condition thereof. Similarly, if the controller comprises a memory, the memory is preferably configured to store the instructions of the processor which allow it to communicate with the control unit.

The user interface may comprise several means for interacting with the user such as a screen, a touch screen, a haptic interface, a microphone, a camera, a button, a keyboard, and an RFID sensor, among others. Furthermore, the user interface may also comprise mobile and/or remote means for interacting, such as one or more Bluetooth antennas, WiFi antennas, among others.

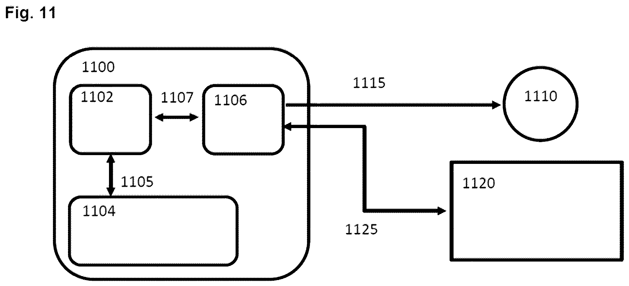

Figure 11 shows a diagram of the interaction between a controller (1100), a solenoid valve (1110), and a user interface (1120). As can be seen, the controller (1100) comprises a processor (1102) and a memory (1104). In other embodiments, the controller (1100) may not comprise the memory (1104). It can also be seen that it is the processor which executes the orders (1115) controlling the solenoid valve, and which sends and receives information (1125) to and from the user interface. Moreover, the memory (1104) sends and stores information (1125) to and from the processor (1102). The processor also sends and receives information and control signals to and from the optical system and/or the electrodes (1107) through the control interface (1106). Likewise, in other embodiments, the processor can also send and receive information to and from an external computer, such as a web server. All these connections can be made by electric or electronic means through cables of any type and/or as telecommunication protocols such as WiFi, Bluetooth, NFC, among others. In other embodiments, it is possible for the controller (1100) to control more than one solenoid valve (1110) and/or user interfaces (1120), or to only control one of the two.

**Claims**

1. A urinary measuring device for taking and measuring urine samples, configured to be arranged in a urinary receptacle; **characterized in that** it comprises:

   a. A funnel (11) for sample collection, comprising a main opening (112);
   b. A urine collection unit (13) connected to the opening for receiving the sample from the funnel and comprising a light reflective surface (732);
   c. A casing (12) for electronics positioned close to the urine collection unit, the casing for electronics containing a light emitter (652) arranged for emitting light on the urine collection unit and a light sensor (654) arranged for measuring the light received by reflection from the urine collection unit
   d. A container (14) for collecting fluid overflowing from the urine collection unit comprising an outlet opening (142), wherein the outlet opening of the container is configured to allow an outflow smaller than that of the main opening (112) of the funnel; and
   e. A plurality of electrodes (16) extending inside the container by means of arms.

2. The urinary measuring device according to claim 1, **characterized in that** the plurality of electrodes are configured to control the light emitter and light sensor.

3. The urinary measuring device according to claim 1 or 2, **characterized in that** the funnel comprises a filter (214) that at least partially covers the main opening configured to prevent direct urination through the main opening.

4. The urinary measuring device according to any of claims 1 to 3, **characterized in that** the light emitter and the sensor are located in the casing for electronics for emitting and receiving light with respect to the urine collection unit such that the planes perpendicular to the emission and measurement directions form at an angle comprised between 120° and 180°.

5. The urinary measuring device according to any of claims 1 to 4, **characterized in that** the casing for electronics comprises a main body, a cover (1021), and a gasket (1024) configured to fluidically seal the interface between the main body and the cover.

6. The urinary measuring device according to any of claims 1 to 5, **characterized in that** the casing for electronics comprises a transparent protector (1055) arranged for protecting the light emitter and the sensor against the sample splashing from the urine collection unit.

7. The urinary measuring device according to any of claims 1 to 6, **characterized in that** the urine collection unit comprises a surface (732) arranged for maximizing the reflection of an incident light and which in turn comprises a material selected from one of the materials from the following list: ceramic, crystal, glass, porcelain, metal, silicone, wood, stones such as marble, polymers such as POM or photosensitive resin, as well as a derivative of these materials.

8. The urinary measuring device according to any of claims 1 to 7, **characterized in that** the device further comprises:

> a voltage source connected between the electrodes configured to apply a voltage signal between the electrodes and a current meter configured to measure the current between the electrodes resulting from a voltage signal applied by the voltage source; or
> an impedance meter (1065) connected to the electrodes, the impedance meter configured to measure the impedance of the fluid present in the container between the electrodes.

9. The urinary measuring device according to any of claims 1 to 8, **characterized in that** the container further comprises a side outlet opening (844), arranged at a greater height than that of the outlet opening and than that of the plurality of electrodes.

10. The urinary measuring device according to any of claims 1 to 9, **characterized by** electric cables extending from the arms to the casing for electronics through one or more gaskets, in order to form an electrical connection between the electrodes and the electronics in the casing for electronics.

11. The urinary measuring device according to any of claims 1 to 10, **characterized in that** the funnel comprises a handle (9125) configured to facilitate the extraction of the urinary measuring device by means of an extraction device.

12. The urinary measuring device according to any of claims 1 to 11, **characterized in that** the casing for electronics further comprises a controller (1026) configured to control the light emitter, the light sensor, and optionally the impedance meter, and wherein the controller is made up of a processor configured to process information from the light emitter, the light sensor, and optionally from the impedance meter.

13. The urinary measuring device according to any of claims 1 to 12, **characterized in that** the urinary measuring device further comprises a valve (1110) configured to control the outflow of water from a water source to the urinary receptacle and wherein the device is configured to control the valve.

14. The urinary measuring device according to claim 13, **characterized in that** the urinary measuring device is configured to open the valve when it detects that sample measurement has ended.

15. The urinary measuring device according to any of claims 1 to 14, **characterized in that** the urinary measuring device further comprises a user interface (1120) configured to receive identification or anonymous information of a user and to receive information about the urinary sample of the user and to show information about the condition of the sample to the user.

**Patentansprüche**

1. Urinmessvorrichtung zum Nehmen und Messen von Urinproben, welche dazu ausgebildet ist, in einem Urinbehältnis angeordnet zu werden; **dadurch gekennzeichnet, dass** sie Folgendes umfasst:

> a. einen Trichter (11) zum Sammeln von Proben, welcher eine Hauptöffnung (112) umfasst;
> b. eine Urinsammeleinheit (13), welche mit der Öffnung zum Erhalten der Probe aus dem Trichter verbunden ist und eine lichtreflektierende Oberfläche (732) umfasst;
> c. ein Gehäuse (12) für elektronische Bauelemente, welches nah an der Urinsammeleinheit positioniert ist, wobei das Gehäuse für elektronische Bauelemente einen Lichtsender (652), welcher zum Senden von Licht auf die Urinsammeleinheit angeordnet ist, und einen Lichtsensor (654), welcher zum Messen des von der Urinsammeleinheit mittels Reflexion erhaltenen Lichtes angeordnet ist, enthält
> d. einen Behälter (14) zum Sammeln des Fluids, welches von der Urinsammeleinheit überfließt, umfassend eine Ausgangsöffnung (142), wobei die Ausgangsöffnung des Behälters dazu ausgebildet ist, einen Ausfluss kleiner als derjenige der Hauptöffnung (112) des Trichters zu erlauben; und

e. eine Vielzahl von Elektroden (16), welche sich innerhalb des Behälters mittels Arme erstrecken.

2. Urinmessvorrichtung nach Anspruch 1, **dadurch gekennzeichnet, dass** die Vielzahl von Elektroden dazu ausgebildet sind, den Lichtsender und den Lichtsensor zu steuern.

3. Urinmessvorrichtung nach Anspruch 1 oder 2, **dadurch gekennzeichnet, dass** der Trichter einen Filter (214) umfasst, welcher die Hauptöffnung mindestens teilweise deckt und dazu ausgebildet ist, ein direktes Urinieren durch die Hauptöffnung zu verhindern.

4. Urinmessvorrichtung nach einem der Ansprüche 1 bis 3, **dadurch gekennzeichnet, dass** sich der Lichtsender und der Sensor im Gehäuse für elektronische Bauelemente befinden, um Licht in Bezug auf die Urinsammeleinheit zu senden und zu erhalten, sodass die Ebenen senkrecht zu den Sende- und Messrichtungen mit einem Winkel, welcher zwischen 120° und 180° liegt, gebildet sind.

5. Urinmessvorrichtung nach einem der Ansprüche 1 bis 4, **dadurch gekennzeichnet, dass** das Gehäuse für elektronische Bauelemente einen Hauptkörper, eine Abdeckung (1021) und eine Dichtung (1024), welche dazu ausgebildet ist, die Trennfläche zwischen dem Hauptkörper und der Abdeckung fluidisch abzudichten, umfasst.

6. Urinmessvorrichtung nach einem der Ansprüche 1 bis 5, **dadurch gekennzeichnet, dass** das Gehäuse für elektronische Bauelemente einen durchsichtigen Schutz (1055) umfasst, welcher zum Schützen des Lichtsenders und des Sensors gegen die von der Urinsammeleinheit spritzende Probe angeordnet ist.

7. Urinmessvorrichtung nach einem der Ansprüche 1 bis 6, **dadurch gekennzeichnet, dass** die Urinsammeleinheit eine Oberfläche (732) umfasst, welche zum Maximieren der Reflexion eines einfallenden Lichtes angeordnet ist, und welche wiederum ein Material umfasst, welches aus einem der Materialien der folgenden Liste ausgewählt wird: Keramik, Kristall, Glas, Porzellan, Metall, Silikon, Holz, Steinen wie Marmor, Polymeren wie POM oder lichtempfindlichem Harz, sowie einem Derivat dieser Materialien.

8. Urinmessvorrichtung nach einem der Ansprüche 1 bis 7, **dadurch gekennzeichnet, dass** die Vorrichtung zusätzlich Folgendes umfasst:

eine zwischen den Elektroden verbundene Spannungsquelle, welche dazu ausgebildet ist, ein Spannungssignal zwischen den Elektroden und einem Strommesser, welcher dazu ausgebildet ist, den Strom zwischen den Elektroden zu messen, welcher sich aus einem von der Spannungsquelle angelegten Spannungssignal ergibt, anzulegen; oder
einen mit den Elektroden verbundenen Impedanzmesser (1065), wobei der Impedanzmesser dazu ausgebildet ist, die Impedanz des im Behälter zwischen den Elektroden vorhandenen Fluids zu messen.

9. Urinmessvorrichtung nach einem der Ansprüche 1 bis 8, **dadurch gekennzeichnet, dass** der Behälter zusätzlich eine seitliche Ausgangsöffnung (844) umfasst, welche auf einer größeren Höhe als diejenige des Ausgangsöffnung und als diejenige der Vielzahl von Elektroden angeordnet ist.

10. Urinmessvorrichtung nach einem der Ansprüche 1 bis 9, **gekennzeichnet durch** Elektrokabel, welche sich von den Armen zum Gehäuse für elektronische Bauelemente durch eine oder mehrere Dichtungen erstrecken, um eine elektrische Verbindung zwischen den Elektroden und den elektronischen Bauelementen im Gehäuse für elektronische Bauelemente zu bilden.

11. Urinmessvorrichtung nach einem der Ansprüche 1 bis 10, **dadurch gekennzeichnet, dass** der Trichter einen Griff (9125) umfasst, welcher dazu ausgebildet ist, die Entnahme der Urinmessvorrichtung mittels einer Entnahmevorrichtung zu erleichtern.

12. Urinmessvorrichtung nach einem der Ansprüche 1 bis 11, **dadurch gekennzeichnet, dass** das Gehäuse für elektronische Bauelemente zusätzlich eine Steuerung (1026) umfasst, welche dazu ausgebildet ist, den Lichtsender, den Lichtsensor und wahlweise den Impedanzmesser zu steuern, und wobei die Steuerung aus einem Prozessor besteht, welcher dazu ausgebildet ist, Information vom Lichtsender, vom Lichtsensor und wahlweise vom Impedanzmesser zu verarbeiten.

13. Urinmessvorrichtung nach einem der Ansprüche 1 bis 12, **dadurch gekennzeichnet, dass** die Urinmessvorrichtung zusätzlich ein Ventil (1110) umfasst, welches dazu ausgebildet ist, den Ausfluss von Wasser von einer Wasserquelle zum Urinbehältnis zu steuern und wobei die Vorrichtung dazu ausgebildet ist, das Ventil zu steuern.

14. Urinmessvorrichtung nach Anspruch 13, **dadurch gekennzeichnet, dass** die Urinmessvorrichtung dazu ausgebildet ist, das Ventil zu öffnen, wenn sie detektiert, dass die Probemessung beendet worden ist.

15. Urinmessvorrichtung nach einem der Ansprüche 1 bis 14, **dadurch gekennzeichnet, dass** die Urinmessvorrichtung zusätzlich eine Benutzerschnittstelle (1120) umfasst, welche dazu ausgebildet ist, Identifizierungsinformation oder anonyme Information eines Benutzers zu erhalten und Information über die Urinprobe des Benutzers zu erhalten und dem Benutzer Information über den Zustand der Probe zu zeigen.

**Revendications**

1. Dispositif de mesure urinaire pour obtenir et mesurer des échantillons d'urine, configuré pour être disposé dans un réservoir urinaire ; **caractérisé en ce qu'**il comprend :

   a. un entonnoir (11) pour le prélèvement d'échantillons, comprenant une ouverture principale (112) ;
   b. une unité de prélèvement d'urine (13) relié à l'ouverture pour recevoir l'échantillon à partir de l'entonnoir et comprenant une surface réfléchissante de la lumière (732) ;
   c. un boîtier (12) pour électronique positionné à proximité de l'unité de prélèvement d'urine, le boîtier pour électronique contenant un émetteur de lumière (652) disposé pour émettre de la lumière sur l'unité de prélèvement d'urine et un capteur de lumière (654) disposé pour mesurer la lumière reçue par réflexion à partir de l'unité de prélèvement d'urine ;
   d. un récipient (14) pour le prélèvement de liquide débordant de l'unité de prélèvement d'urine comprenant une ouverture de sortie (142), dans lequel l'ouverture de sortie du récipient est configurée pour permettre un écoulement de sortie plus petit que celui de l'ouverture principale (112) de l'entonnoir ; et
   e. une pluralité d'électrodes (16) s'étendant à l'intérieur du récipient par le biais de bras.

2. Dispositif de mesure urinaire selon la revendication 1, **caractérisé en ce que** la pluralité d'électrodes sont configurés pour contrôler l'émetteur de lumière et le capteur de lumière.

3. Dispositif de mesure urinaire selon la revendication 1 ou 2, **caractérisé en ce que** l'entonnoir comprend un filtre (214) qui recouvre au moins partiellement l'ouverture principale, configuré pour empêcher une miction directe à travers l'ouverture principale.

4. Dispositif de mesure urinaire selon l'une quelconque des revendications 1 à 3, **caractérisé en ce que** l'émetteur de lumière et le capteur sont situés dans le boîtier pour électronique pour émettre et recevoir de la lumière par rapport à l'unité de prélèvement d'urine de telle sorte que les plans perpendiculaires aux directions d'émission et de mesure forment un angle compris entre 120° et 180°.

5. Dispositif de mesure urinaire selon l'une quelconque des revendications 1 à 4, **caractérisé en ce que** le boîtier pour électronique comprend un corps principal, un couvercle (1021) et un joint (1024) configuré pour sceller de manière fluidique l'interface entre le corps principal et le couvercle.

6. Dispositif de mesure urinaire selon l'une quelconque des revendications 1 à 5, **caractérisé en ce que** le boîtier pour électronique comprend un protecteur transparent (1055) disposé pour protéger l'émetteur de lumière et le capteur contre les éclaboussures d'échantillon à partir de l'unité de prélèvement d'urine.

7. Dispositif de mesure urinaire selon l'une quelconque des revendications 1 à 6, **caractérisé en ce que** l'unité de prélèvement d'urine comprend une surface (732) disposée pour maximiser la réflexion d'une lumière incidente et qui comprend à son tour un matériau choisi parmi l'un des matériaux de la liste suivante : de la céramique, du cristal, du verre, de la porcelaine, du métal, de la silicone, du bois, des pierres telles que le marbre, des polymères tels que POM ou de la résine photosensible, ainsi qu'un dérivé de ces matériaux.

8. Dispositif de mesure urinaire selon l'une quelconque des revendications 1 à 7, **caractérisé en ce que** le dispositif comprend en outre :

une source de tension reliée entre les électrodes configurée pour appliquer un signal de tension entre les électrodes et un ampèremètre configuré pour mesurer le courant entre les électrodes résultant d'un signal de tension appliqué par la source de tension ; ou

un impédancemètre (1065) relié aux électrodes, l'impédancemètre étant configuré pour mesurer l'impédance du liquide présent dans le récipient entre les électrodes.

9. Dispositif de mesure urinaire selon l'une quelconque des revendications 1 à 8, **caractérisé en ce que** le récipient comprend en outre une ouverture de sortie latérale (844), disposée à une hauteur supérieure à celle de l'ouverture de sortie et celle de la pluralité d'électrodes.

10. Dispositif de mesure urinaire selon l'une quelconque des revendications 1 à 9, **caractérisé par** des câbles électriques s'étendant des bras jusqu'au boîtier pour électronique à travers un ou plusieurs joints, afin de former une connexion électrique entre les électrodes et l'électronique dans le boîtier pour électronique.

11. Dispositif de mesure urinaire selon l'une quelconque des revendications 1 à 10, **caractérisé en ce que** l'entonnoir comprend une poignée (9125) configurée pour faciliter l'extraction du dispositif de mesure urinaire par le biais d'un dispositif d'extraction.

12. Dispositif de mesure urinaire selon l'une quelconque des revendications 1 à 11, **caractérisé en ce que** le boîtier pour électronique comprend en outre un contrôleur (1026) configuré pour contrôler l'émetteur de lumière, le capteur de lumière, et optionnellement l'impédancemètre, et dans lequel le contrôleur est constitué d'un processeur configuré pour traiter des informations provenant de l'émetteur de lumière, du capteur de lumière, et optionnellement de l'impédancemètre.

13. Dispositif de mesure urinaire selon l'une quelconque des revendications 1 à 12, **caractérisé en ce que** le dispositif de mesure urinaire comprend en outre une vanne (1110) configurée pour contrôler l'écoulement de sortie d'eau à partir d'une source d'eau jusqu'au réservoir urinaire et dans lequel le dispositif est configuré pour contrôler la vanne.

14. Dispositif de mesure urinaire selon la revendication 13, **caractérisé en ce que** le dispositif de mesure urinaire est configuré pour ouvrir la vanne lorsqu'il détecte que la mesure de l'échantillon est terminée.

15. Dispositif de mesure urinaire selon l'une quelconque des revendications 1 à 14, **caractérisé en ce que** le dispositif de mesure urinaire comprend en outre une interface utilisateur (1120) configurée pour recevoir des informations d'identification ou anonymes d'un utilisateur et pour recevoir des informations concernant l'échantillon urinaire de l'utilisateur et pour montrer à l'utilisateur des informations concernant l'état de l'échantillon.

FIG 1A

10

11

112

111

14

FIG 1B

10

11

12

15

14

16

142

13

112

FIG 2A

214

2141

FIG 2B

214

2141

FIG 2C

21

214

212

FIG 3A

37

371
372
373
31

313

312

FIG 3B

37

312

31

FIG 4A

41

412
416

FIG 4B

41

416

412

417

FIG 4C

40

412

416

417

44

FIG 5A

51

512

518

516

FIG 5B

51

512

518

FIG 6A

FIG 6B

FIG 6C

652    65    654

65
652    654

62
65

FIG 7A

FIG 7B

FIG 7C

734    73    732
734
734

73
734

72
7!
734    734
73

FIG 7D

732
752    754
73

EP 4 462 119 B1

FIG 8A          FIG 8B          FIG 8C

FIG 9A          FIG 9B

**Fig. 10**

1000

1014

107

101

1021

1024

1026

1022

105

1055

103

1064

106

1062

104

Fig. 11

**Fig. 12**

**FIG. 13**

**FIG. 14**

**FIG 15A**

**FIG 15B**

$y = -1003.3x + 5822.7$
$R^2 = 0.8957$

**FIG 15C**

**FIG 15D**

$y = -976.54x + 10210$
$R^2 = 0.9788$

**FIG 15E**

**FIG 15F**

$y = -2066.3x + 14021$
$R^2 = 0.9615$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015196254 A